# EUROPEAN PATENT APPLICATION

(11) **EP 4 811 373 A1**
(43) Date of publication of application: **23.09.2026**
(21) Application number: 26165451.1
(22) Date of filing: 17.03.2026
(51) Int. Cl.: G16H 20/40

(54) **RADIOTHERAPY GRAPHICAL USER INTERFACES**

(30) Priority: 21.03.2025 US 202519087365
(71) Applicant: Varian Medical Systems, Inc., Palo Alto, CA 94304 (US)
(72) Inventor: SPATOLA, Brian, Palo Alto, 94304 (US); VOJAN, Filip, Palo Alto, 94304 (US)
(74) Representative: Mathisen & Macara LLP

(57) **Abstract**

Embodiments describe dynamically managing and presenting task-specific instructions of a radiotherapy treatment on a display screen (226) integrated into the gantry (224) of a radiotherapy machine (241). One method (300) includes presenting (310) a set of icons (406-432) corresponding to instructions associated with the radiotherapy treatment. Upon detecting (320) interaction with an icon (406-432), the processor displays (320) a subset of instructions relevant to the selected task on the screen (226). The method (300) further involves presenting (330) a visual indicator (440) to signal that the subset of instructions satisfies a predefined threshold and is distributed across multiple pages. In response (340) to a second interaction with the visual indicator (440), the processor transitions from a first page to a second page, enabling seamless navigation. This approach ensures an intuitive, task-oriented interface that enhances workflow efficiency, reduces cognitive load, and provides operators with tailored, real-time guidance for delivering precise and effective radiotherapy treatments.

## Description

### TECHNICAL FIELD

This application relates generally to graphical user interfaces and software for radiotherapy treatment setup and machines.

### BACKGROUND

Radiation therapy, which utilizes ionizing radiation, is a localized treatment targeting specific tissues, such as cancerous tumors. Ideally, this therapy focuses on the planning target volume (PTV) or target organ, sparing surrounding healthy tissue from excessive radiation doses to minimize damage. To ensure the prescribed dose is accurately delivered to the PTV, the patient must be precisely positioned relative to the linear accelerator, typically using a movable treatment couch on a turntable assembly. Implementing radiation therapy is a complex process involving specific guidelines, protocols, and instructions followed by various medical professionals, including clinicians, technicians, device manufacturers, and treating physicians. Due to the hazards associated with radiation, it is crucial that all instructions are meticulously followed.

Conventional radiation therapy systems are often complex to operate, requiring extensive training for efficient workflow execution. Tasks such as patient positioning, system calibration, and machine adjustments involve numerous interactions with both the patient and the radiotherapy machine. The data needed for these setups is often voluminous and complex, typically displayed on a screen within the treatment room. However, these conventional methods can be inefficient, ineffective, or even dangerous. Conventional radiotherapy systems do not efficiently display the data needed for patient and machine setup. In some conventional systems and methods, the necessary information for an operator (e.g., radiotherapy technician or any other medical professional) is not presented in a manner that is intuitive and user-friendly, interrupting the adjustment process and proving to be ineffective and time-consuming. Some other conventional systems and methods often provide all the information via static and complex graphical user interfaces (GUIs). Unless an operator is highly experienced with a particular system, this can slow down the setup process and degrade the patient's experience.

### SUMMARY

In accordance with a first aspect of the invention, there is provided a computer-readable medium as defined by claim 1.

In accordance with a second aspect of the invention, there is provided a system as defined by claim 6.

In accordance with a third aspect of the invention, there is provided a method as defined by claim 11.

Optional features are defined by the dependent claims.

Given the complexities involved and discussed herein, there is a need for an intuitive and workflow-oriented graphical user interface (GUI) specific to radiotherapy treatments that efficiently displays treatment setup data, such as machine parameters, patient alignment data, and necessary accessories.

The methods and systems discussed herein provide a streamlined and intuitive approach to consuming radiotherapy information within a single page while ensuring that all other relevant treatment data remains readily accessible. By dynamically updating a portion of the display screen (e.g., central portion) based on interactions with icons, the methods and systems discussed herein allow operators to focus on task-specific details without needing to navigate away or switch screens. In some embodiments, the interaction with the icons is indirect, as users operate the controls (e.g., buttons) on the hand pendant rather than directly interacting with the icons displayed on the screen. By using the hand pendant, operators can efficiently navigate through screens, adjust parameters, and confirm alignment. This approach integrates task-relevant instructions, visual indicators, and real-time updates within the same interface, reducing the cognitive load and enabling operators to maintain continuity during critical workflows. Additionally, the presence of radially arranged icons ensures that the broader context of the treatment process-such as alignment, calibration, or dose delivery-is always visible and accessible, providing a comprehensive view without cluttering the interface. This unified design significantly enhances usability and ensures that operators can efficiently manage complex radiotherapy procedures while maintaining access to all necessary information.

In one embodiment, the techniques described herein relate to a computer-readable medium having non-transitory instructions, that when executed, cause a processor to: present, on a display screen located on a gantry of a radiotherapy machine configured to implement radiotherapy treatment of a patient, a set of icons corresponding to a set of instructions associated with the radiotherapy treatment; in response to determining an interaction with at least one icon of the set of icons, display on the display screen a subset of the set of instructions corresponding to the at least one icon; present a visual indicator on the display screen indicating that the subset of the set of instructions satisfies a threshold and is displayed in a plurality of pages; and in response to determining a second interaction with the visual indicator, transition from a first page of the plurality of pages to a second page of the plurality of pages while displaying the set of icons on both the first page and the second page.

The threshold may correspond to a number of instructions of the subset of the set of instructions.

The threshold may correspond to a sequential order of the subset of the set of instructions.

The visual indicator may convey a number of pages of the plurality of pages.

The second interaction may indicate a particular page of the plurality of pages being displayed.

The instructions may, when executed, cause the processor to revise a visual attribute of the at least one icon.

The first page may display a different category of radiotherapy data than the second page.

In another embodiment, the techniques described herein relate to a system including: a radiotherapy machine having a display screen; and at least one processor in communication with the display screen, the at least one processor configured to: present, on the display screen located on a gantry of the radiotherapy machine configured to implement radiotherapy treatment of a patient, a set of icons corresponding to a set of instructions associated with the radiotherapy treatment; in response to determining an interaction with at least one icon of the set of icons, display on the display screen a subset of the set of instructions corresponding to the at least one icon; present a visual indicator on the display screen indicating that the subset of the set of instructions satisfies a threshold and is displayed in a plurality of pages; and in response to determining a second interaction with the visual indicator, transition from a first page of the plurality of pages to a second page of the plurality of pages while displaying the set of icons on both the first page and the second page.

The threshold may correspond to a number of instructions of the subset of the set of instructions.

The threshold may correspond to a sequential order of the subset of the set of instructions.

The visual indicator may convey a number of pages of the plurality of pages.

The second interaction may indicate a particular page of the plurality of pages being displayed.

The processor may be further configured to revise a visual attribute of the at least one icon.

The first page may display a different category of radiotherapy data than the second page.

In yet another embodiment, the techniques described herein relate to a method including: presenting, by at least one processor on a display screen located on a gantry of a radiotherapy machine configured to implement radiotherapy treatment of a patient, a set of icons corresponding to a set of instructions associated with the radiotherapy treatment; in response to determining an interaction with at least one icon of the set of icons, displaying, by the at least one processor, on the display screen a subset of the set of instructions corresponding to the at least one icon; presenting, by the at least one processor, a visual indicator on the display screen indicating that the subset of the set of instructions satisfies a threshold and is displayed in a plurality of pages; and in response to determining a second interaction with the visual indicator, transitioning, by the at least one processor, from a first page of the plurality of pages to a second page of the plurality of pages while displaying the set of icons on both the first page and the second page.

The threshold may correspond to a number of instructions of the subset of the set of instructions.

The threshold may correspond to a sequential order of the subset of the set of instructions.

The visual indicator may convey a number of pages of the plurality of pages.

The second interaction may indicate a particular page of the plurality of pages being displayed.

The method may further include revising a visual attribute of the at least one icon.

The first page may display a different category of radiotherapy data than the second page.

The methods discussed herein may assist the user in performing a radiotherapy setup workflow, by means of a continued and/or guided process of human-machine interaction. The systems discussed herein may be configured to assist the user in performing a radiotherapy workflow and/or a radiotherapy setup workflow, by means of a continued and/or guided process of human-machine interaction.

### BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.
**FIG. 1** illustrates components of a workflow-oriented radiotherapy system, according to an embodiment.
**FIG. 2A** illustrates a radiotherapy machine in a default, upright orientation, according to an embodiment.
**FIG. 2B** illustrates a radiotherapy machine in a rotated orientation, according to an embodiment.
**FIG. 3** is a flow diagram of a method for displaying radiotherapy icons and information on a radiotherapy, according to an embodiment.
**FIGS. 4A-4C** illustrate a radiotherapy machine displaying a set of icons and information, according to an embodiment.
**FIGS. 5A-5B** illustrate a graphical user interface displayed on a radiotherapy machine, according to an embodiment.
**FIG. 6** illustrates a graphical user interface displayed on a radiotherapy machine, according to an embodiment.

### DETAILED DESCRIPTION

Reference will now be made to some embodiments illustrated in the drawings, and specific language will be used here to describe the same. It will nevertheless be understood that no limitation of the scope of the embodiments of the methods and systems described herein is thereby intended. Alterations and further modifications of the features illustrated here, and additional applications of the principles of the embodiments of the methods and systems described herein as illustrated here, which would occur to a person skilled in the relevant art and having possession of this disclosure, are to be considered within the scope of the embodiments, methods, and/or systems described herein.

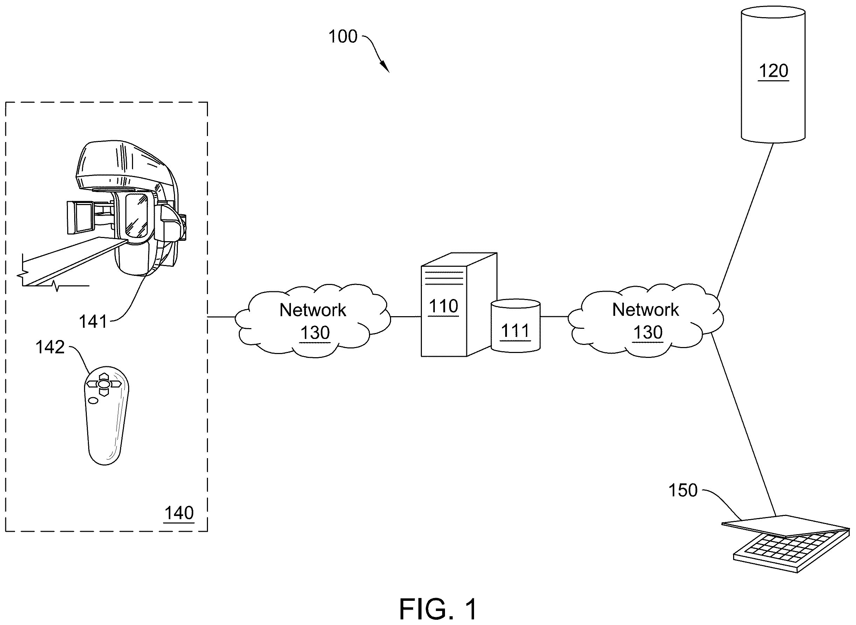

The methods described herein can be implemented using various computing devices/features described in **FIG. 1****.** Therefore, **FIG. 1** describes a non-limiting example of a computer environment where a server can perform the processes/methods described herein, such as retrieving, processing, and presenting radiotherapy treatment data, and presenting data for a graphical user interface (GUI) having workflow-oriented pages (or instances) on various displays screens.

**FIG. 1** illustrates various components of a system **100** for presenting various pages related to operation of a radiotherapy treatment, in accordance with an embodiment. The system **100** may include an analytics server **110,** a medical records database **120,** a radiotherapy system **140,** and a system administrator computer **150** or workstation. These features may communicate with each other over a network **130.** For example, the system **100** may present, optionally via the analytics server **110,** one or more pages/GUIs on the radiotherapy machine **141.** In another example, the system **100** may present, optionally via the analytics server **110,** a page presenting a live feed of a patient on one or more display devices, such as the display device on a gantry of the radiotherapy machine **141.** In general, any of the steps discussed herein as performed by the analytics server may be performed by the system in general.

The network **130** may include wired and/or wireless communications according to one or more standards via one or more transport mediums. Communication over the network **130** may be in accordance with various communication protocols, such as transmission control protocol and internet protocol (TCP/IP), user datagram protocol (UDP), and Institute of Electrical and Electronics Engineers (IEEE) communication protocols. The network **130** may further include wireless communications according to Bluetooth specification sets, or another standard or proprietary wireless communication protocol. The network **130** may further include communications over a cellular network, including, for example, a global system for mobile (GSM) communications, code division multiple access (CDMA), and enhanced data for global evolution network (EDGE). The examples of the network **130** may include, but are not limited to, private or public local area network (LAN), wireless LAN (WLAN), metropolitan area network (MAN), wide area network (WAN), and the Internet.

The analytics server **110** may be any computing device capable of performing the actions described herein. For instance, the analytics server **110** includes a processing unit and a non-transitory machine-readable storage medium. The processing unit includes a processor with a computer-readable medium, such as a random-access memory coupled to the processor. In some embodiments, the analytics server **110** executes algorithms or computer executable program instructions, which may be executed by a single processor or multiple processors in a distributed configuration. The instructions allow the processor to implement the functionality described herein. The analytics server **110** may be configured to interact with one or more software modules of a same or a different type operating within the system **100.**

Non-limiting examples of the processor may include a microprocessor, an application specific integrated circuit, and a field programmable object array, among others. The analytics server **110** may be capable of executing data processing tasks, data analysis tasks, and valuation tasks. Non-limiting examples of the analytics server **110** may include a desktop computer, a server computer, a laptop computer, a tablet computer, and the like. For simplicity, **FIG. 1** depicts a single-server computing device functioning as the analytics server **110.** However, some embodiments may include a plurality of server computing devices capable of performing various tasks described herein.

Implementation of the methods described herein is not limited to the system architecture depicted in **FIG. 1****.** In alternative embodiments, the analytics server **110** may be an embedded computing device disposed within the radiotherapy machine **141.** In some embodiments, the analytics server **110** may be a plurality of computing devices operated locally and/or remotely. In various embodiments, the analytics server **110** may be operated through a cloud service (e.g., network, internet). The cloud service may be performed in accordance with various communication protocols such as TCP/IP, UDP, and IEEE communication protocols. The analytics server **110** may utilize a database, such as a local database **111,** to store and/or retrieve various data described herein. For instance, the analytics server **110** may store different data corresponding to the different pages within the local database **111.** The page may be displayed on a display screen associated with the radiotherapy system **140.**

For instance, the analytics server **110** may display a page having a live feed of the patient before the treatment has begun and/or may display various pages describing how to position the patient on the couch and/or how to adjust the radiotherapy machine **141.** Even though some embodiments describe the analytics server **110** displaying various pages on a display screen attached to the radiotherapy machine **141** (e.g., located on the gantry of the radiotherapy machine **141**), it is expressly understood that the analytics server **110** may display different pages described herein on a display screen located anywhere within the treatment room, such as located on the wall of the treatment room or on any electronic device within the treatment room. In some configurations, the analytics server **110** may display the pages on the system administrator computer **150** or workstation.

The local database **111** may also store data corresponding to the radiotherapy machine **141** (e.g., default position of the radiotherapy machine **141,** and/or current position of the radiotherapy machine **141,** such as the orientation of the bed/couch and/or gantry).

If the analytics server **110** receives a request from the radiotherapy system **140** to provide the current position/orientation of the radiotherapy machine **141,** the analytics server **110** may query the local database **111** and may retrieve the corresponding data. Additionally, or alternatively, the analytics server **110** may also communicate with various sensors associated with the radiotherapy system **140** to retrieve machine data and parameters. The analytics server **110** may then use the methods/systems described herein to instruct the radiotherapy machine **141** to adjust the positioning of the radiotherapy machine **141** to the default position of the radiotherapy machine **141** or another position of the radiotherapy machine, or to display one or more pages instructing a technician to adjust the radiotherapy machine **141** and/or the patient.

The local database **111** may also store data associated with different users of the radiotherapy system **140.** In a non-limiting example, the user data may include the user's login (e.g., sign-in, credentials) information and authorization levels. For example, the analytics server **110** may store, using the local database **111,** the user's name and password. The analytics server **110** may allow the user to log in to the radiotherapy machine **141,** and/or the system administrator computer **150.** If the user logs-in into the system, the analytics server **110** may adjust what the user is able to view, adjust, and control based on the user's authorization level. The analytics server **110** may conceal the patient's medical information that is not relevant to the radiation treatment from the medical technician. In another example, the analytics server **110** may conceal all of the patient's medical information if a machine technician signs-in.

The analytics server **110** may also utilize one or more other databases, such as the medical records database **120,** to store and/or retrieve various data described herein. The databases herein can be configured as one or more databases storing the data, and the disclosure is not intended to be limited to a particular number or location of databases. The analytics server **110** may instruct the medical records database **120** to store patient data (e.g., patient name, patient machine alignment information) associated with a patient identifier (e.g., patient's name, patient's profile image). The analytics server **110** may then instruct the medical records database **120** to populate a dataset corresponding to a patient and display the profile image of the patient. If the analytics server **110** receives a request from the radiotherapy system **140** to display the data associated with the patient identifier, the analytics server **110** may query the medical records database **120** and may retrieve the corresponding dataset. The analytics server **110** may then use the methods/systems described herein to dynamically display the patient data in accordance with the patient identifier.

The medical records database **120** may also include a radiotherapy treatment file associated with the patient identifier. As used herein, the radiotherapy treatment file refers to all data associated with a patient's radiation therapy treatment and is not limited to a particular step or information. The radiotherapy treatment file may also be retrieved from the radiotherapy system **140,** the medical records database **120,** and/or the system administrator computer **150.** The radiotherapy treatment file may include the treatment data associated with a patient. The radiotherapy treatment file may be associated with a patient identifier and may also be updated after a patient has completed treatment. For example, after the patient has completed a treatment session, the medical records database **120** may retrieve the duration of the treatment session from the radiotherapy system **140** and update the radiotherapy file to include the duration of the treatment session. Even though aspects of the embodiments described herein discuss radiotherapy treatment as a file, the radiotherapy treatment file represents a collection of the patient's medical and treatment data, which may be stored in different files. For brevity, the present disclosure refers to the patient's data as a radiotherapy treatment file.

The radiotherapy treatment file may include data for treatment plans for one or more patients where each treatment plan is specific to a single patient. For instance, each treatment plan is uniquely created for each patient and corresponds to the patient's unique attributes (e.g., physical attributes of the patient and the patient's unique condition to be treated). In some configurations, each treatment plan for each patient may itself include multiple files.

The analytics server **110** may retrieve treatment data associated with a patient that is stored within the patient's radiotherapy treatment file(s). As described herein, the analytics server **110** may analyze the treatment data and may display various features and graphical components described herein. While the medical records database **120** may contain treatment data (radiotherapy treatment files) associated with multiple patients, the methods described herein are implemented such that various pages and graphical features described herein are specific to the particular patient being treated.

In some configurations, the analytics server **110** may retrieve radiotherapy treatment files associated with multiple patients. The analytics server **110** may then receive a selection by an operator (e.g., technician) of a patient to be treated. As a result, the analytics server **110** identifies the radiotherapy treatment file associated with the selected patient and customizes the graphical components described herein for the selected patient.

The analytics server **110** may also retrieve and instruct the medical records database **120** to store patient data associated with the patient from the medical records database **120,** the radiotherapy system **140,** and/or the system administrator computer **150.** For instance, the medical records database **120** may include patient data (e.g., previously populated by the analytics server **110** and/or periodically retrieved from a third-party data source). If a patient is selected, the analytics server **110** may query and retrieve patient data from the medical records database **120** and provide the retrieved patient data. For instance, the analytics server **110** may display the patient's profile image when the patient is selected, providing an opportunity to verify the correct patient was selected.

The analytics server **110** may receive treatment data associated with a patient from the medical records database **120,** the treatment data may further include at least a patient identifier. The analytics server may receive radiotherapy treatment file associated with one or more patients from the medical records database **120.** The radiotherapy treatment file may refer to a file having data associated with a process in which a medical team (e.g., radiation oncologists, radiation therapist, medical physicists, and/or medical dosimetrists) plan the appropriate external beam radiotherapy or internal brachytherapy treatment techniques for a patient.

The data within the radiotherapy file is not limited to the external radiation therapy as other treatments may impact the radiation therapy, such as medical oncology treatment (chemotherapy), interventional oncology treatment (e.g. cryotherapy, microwave therapy, or embolic therapy) or other non-treatment procedures, such as labs and appointments with other medical professionals. The radiotherapy treatment file may include data specific to one or more patient's radiotherapy treatment. The radiotherapy treatment file may include any combination of one or more of: a patient identifier, patient's electronic health data records, medical images (e.g., CT scans, 4D CT Scans, MRIs, and x-ray images), treatment-specific data (e.g., arc information or treatment type), target organ (e.g., specification and location data to identify the tumor to be eradicated), treatment plan, etc. Additional examples may include non-target organs, dosage-related calculations (e.g., radiation dose distribution within an anatomical region of the patient), and/or radiotherapy machine specific information (e.g., couch-gantry orientations, machine trajectory, control points, dose distributions, and/or arc information).

The analytics server **110** may use the patient identifier within the radiotherapy treatment file to identify a particular patient and retrieve additional information regarding said patient. For instance, the analytics server **110** may query the medical records database **120** to identify medical data associated with the patient. For instance, the analytics server may query data associated with the patient's anatomy, such as physical data (e.g., height, weight, and/or body mass index) and/or other health-related data (e.g., blood pressure or other data relevant to the patient receiving radiotherapy treatment). The analytics server **110** may also retrieve data associated with current and/or previous medical treatments received by the patient (e.g., prior treatment fractions, or data associated with the patient's previous surgeries).

The analytics server **110** may analyze the data received and generate additional queries accordingly. For instance, the analytics server **110** may retrieve data associated with one or more medical (or other) devices needed for the patient. The analytics server may retrieve data indicating that the patient suffers from a respiratory medical condition. As a result, the analytics server **110** may generate and transmit a query to the radiotherapy machine **141,** or the system administrator computer **150** to identify whether the patient uses/needs a ventilator.

If necessary, the analytics server **110** may also analyze the patient's medical data records to identify the needed patient attributes. For instance, the analytics server **110** may query a database to identify the patient's BMI. However, because many medical records are not digitalized, the analytics server **110** may not receive the patient's BMI value using simple query techniques. As a result, the analytics server **110** may retrieve the patient's electronic health data and may execute one or more analytical protocols (e.g., natural language processing) to identify the patient's body mass index. In another example, if the analytics server **110** does not receive tumor data (e.g., end-points) the analytics server **110** may execute various image recognition protocols and identify the tumor data.

Another example of a patient attribute may include specific tumor locations. More specifically, this data may indicate the primary tumor location with respect to the patient's centerline. This data may be inputted by the treating oncologist or may be analyzed using various image recognition or segmentation methods executed on the patient's medical images.

Another example of a patient attribute may include whether the patient uses prosthesis (e.g., hip or femoral head prosthesis). This attribute may result in a change of the patient's treatment (e.g., patients with these conditions might require a special treatment).

The analytics server **110** may use various application-programming interfaces (APIs) to communicate with different features described herein. As used herein, an API refers to a computing interface that uses connector programming code to act as a software intermediary between at least two computing components/features described herein. The API may automatically and/or periodically transfer various calls, instructions, and/or requests among different features of the system **100.** Using different APIs, the analytics server **110** may automatically transmit and/or receive calls and instructions.

Additionally, or alternatively, the analytics server **110** may use a content delivery network (CDN) to ensure data integrity when communicating with different features described in the system **100.** As described herein, a CDN refers to a distributed delivery network of proxy servers/nodes that uses multi-layered delivery methods/systems to transmit data (e.g., Akamai). The analytics server **110** may use a CDN when communicating various calls/instructions with the network **130** and/or the local database **111.**

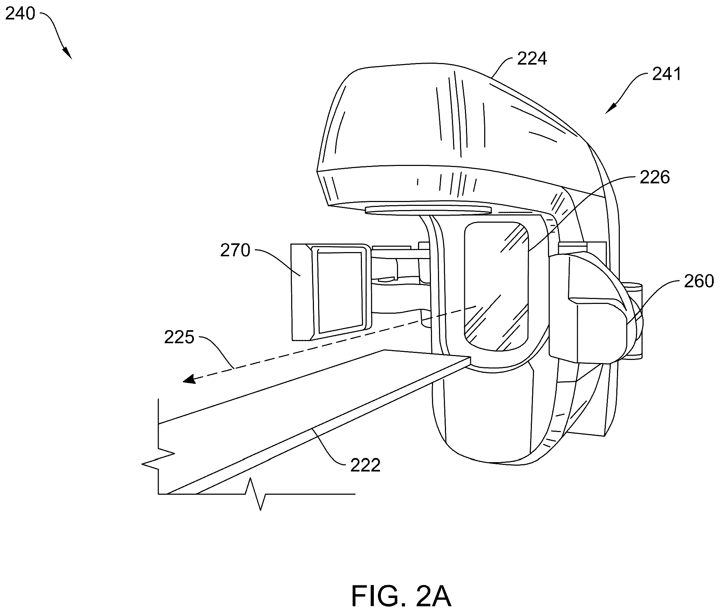

The radiotherapy machine **141** may also include a couch (shown as couch **222** in **FIG. 2A**) to align the patient in a designated position before the treatment begins. The designated position may be identified, calculated, and/or retrieved by the analytics server **110.** For example, the analytics server **110** may retrieve patient data from the medical records database **120,** analyze the data, and utilize the analyzed data to position the patient on the couch. In some embodiments, the doctor identifies how to align the patient on the couch to optimize the treatment therapy. Optionally, the radiotherapy machine **141** directs radiation at specified locations of the patient resting on the couch during treatment. The specified locations may be selected by the technician operating the radiotherapy machine **141,** the pendant **142,** and/or the system administrator computer **150.** The analytics server **110** may also specify the locations on the patient to direct the radiation.

The radiotherapy machine **141** may also include an x-ray emitter and an x-ray receiver. The x-ray emitter may be disposed on a gantry of the radiotherapy machine **141** and may be configured to provide x-ray (e.g., a penetrating form of high-energy electromagnetic radiation) waves. The x-ray emitter emits x-ray waves to the patient who is positioned on the couch. The x-ray receiver may be disposed opposed to the x-ray emitter. The x-ray waves received by the x-ray receiver generate an imprint (e.g., image) of the patient's internal anatomy. Although the example embodiment recites the use of x-ray imaging, an alternative configuration for the radiotherapy machine may include an additional or other medical imaging apparatus (e.g., fluoroscopy apparatus, MRI, etc.).

The radiotherapy machine **141** may also include a set of cameras (e.g., camera on an end of the couch, gantry camera, ceiling camera, or other cameras placed within the radiotherapy room). The analytics server **110** may retrieve a live feed of the couch (e.g., with or without the patient) from the set of cameras and provide it to a display screen disposed on the radiotherapy machine **141.** The set of cameras may also directly communicate with the radiotherapy system **140.**

The radiotherapy machine **141** may also include a gantry that may be in communication with the analytics server **110.** If the radiotherapy machine **141** is in operation, the gantry may rotate relative to the radiotherapy machine **141** to begin the treatment of the patient. The analytics server **110** may use the live feed of the set of cameras to control and/or stop the movement of the gantry. During operation, the analytics server **110** may instruct the display screen of the radiotherapy machine **141** to display the live feed of the set of cameras and/or a designated page to the display screen of the radiotherapy machine **141.** In various embodiments, the analytics server **110** may not instruct the display screen of the radiotherapy machine **141** to activate the display screen of the radiotherapy machine **141** during operation. In some embodiments, the display screen of the radiotherapy machine **141** may be a touch screen and may control the pages generated by the analytics server **110** through the display screen of the radiotherapy machine **141.**

The radiotherapy machine **141** may have a default home position. Before the patient receives treatment, the radiotherapy machine **141** may be reset to a position that is ergonomically desirable for the patient. The default home position may also be optimized to receive a new patient or allow the patient to more easily get off the couch.

As discussed in greater detail herein, the radiotherapy machine **141** may also be controlled by the pendant **142.** The pendant **142** may be connected to the radiotherapy machine **141** through wired or wireless communications according to, for example, Bluetooth specification sets or another standard or proprietary wireless communication protocol. In various embodiments, the pendant **142** may be connected to the radiotherapy machine **141** through a wired connection or be integrated into the radiotherapy machine **141.** The pendant **142** may also be in communication with the analytics server **110.** The pendant **142** may adjust the positioning of the radiotherapy machine **141** through a selection of buttons that axially actuates the radiotherapy machine **141** (e.g., couch) towards a desired direction. The technician may also use the pendant **142** to initialize the radiotherapy machine **141,** and/or to have the radiotherapy machine **141** return to its home position.

The pendant **142** may also allow the technician to input patient information, which may be then communicated to the radiotherapy machine **141,** the analytics server **110,** and/or the medical records database **120.** The pendant **142** may be used to control one or more of the steps of the setup and/or treatment of the patient.

The pendant **142** may also include a touch pad (e.g., tactile sensor). The touch pad may be used to control various pages generated by the analytics server **110.** For example, the user may use the touch pad of the pendant **142** to control a page, generated by the analytics server **110,** to proceed through the workflow steps of the radiotherapy setup and/or treatment. In some embodiments, the page may be controlled through a touch screen on the radiotherapy machine **141** or elsewhere in the treatment room.

As discussed in greater detail herein, the radiotherapy system **140** may further include accessories that assist in the radiotherapy setup and/or treatment of the patient. The analytics server **110** may communicate with the radiotherapy system **140** to select which accessories are required. The analytics server **110** may also communicate with the medical records database **120,** and/or the system administrator computer **150** to determine which accessories are required. For example, a patient may require a specific accessory for their radiotherapy setup and/or treatment. The analytics server **110** may retrieve that the patient requires a specific accessory by accessing the medical records database **120** and communicate that information to the radiotherapy system **140.** The accessories may be in communication with the radiotherapy system **140.** For instance, as will be described below, the analytics server may use RFID tags to scan and identify the location and/or presence of various accessories. In some embodiments, the accessories are wired or otherwise integrated into the radiotherapy machine **141.**

The local database **111** associated with the analytics server **110,** the medical records database **120,** and the radiotherapy machine **141** are capable of storing information in various formats and/or encrypted versions. The information may include data records associated with various patient information, user preferences, a set of prompts (e.g., question, query, and inquiry), attributes associated with various pages to be generated by the analytics server **110,** and the like. The medical records database **120,** may have a logical construct of data files, which are stored in non-transitory machine-readable storage media, such as a hard disk or memory, controlled by software modules of a database program (e.g., structured query language (SQL)), and a database management system that executes the code modules (e.g., SQL scripts) for various data queries and management functions.

The system administrator computer **150** may represent a computing device operated by a system administrator. The system administrator computer **150** may communicate with the analytics server **110.** The system administrator computer **150** may be configured to display various analytic metrics where the system administrator can monitor gantry movement, patient information, and modify various thresholds/rules described herein. The system administrator computer **150** may be configurable to display certain analytics metrics when specific thresholds/rules have been exceeded. For example, the system administrator computer **150** may be alerted if a patient has moved a specified amount during treatment. The analytics server **110** may allow the system administrator computer **150** to also control and/or override the settings of the radiotherapy machine **141,** and/or the pendant **142.** For instance, an administrator may revise the minimum distance threshold and/or customize any feature on the pages described herein (e.g., move features within the page, color, font, shape, size, or the order of display for one or more pages).

The analytics server **110** may configure the system administrator computer **150** to review any and/or all commands made through the radiotherapy system **140** at specified process steps. The system administrator computer **150** may then allow or reject the commands made through the radiotherapy system **140.** For example, before the technician starts the patient treatment using the radiotherapy machine **141,** the analytics server **110** may first prompt the system administrator computer **150** to review the radiotherapy machine **141** parameters for approval. Once approved, the technician may then control the radiotherapy machine **141.**

The analytics server **110** may configure the system administrator computer **150** to review any and/or all reading and/or (over)writing of patient data to and/or from the medical records database **120.** For example, before the technician may (over)write patient information collected by the radiotherapy machine **141,** the analytics server **110** may first prompt the system administrator computer **150** to review the patient information before it is stored in the medical records database **120.**

**FIG. 2A** illustrates, as described above, a radiotherapy system **240,** including a radiotherapy machine **241** that includes a gantry **224** that rotates in a direction **228** (which may be clockwise or counterclockwise) about a center axis **225** of the radiotherapy machine **241.** In some embodiments, the radiotherapy system **240** may be substantially similar to the radiotherapy system **140** of **FIG. 1****.** Likewise, the radiotherapy machine **241** may be substantially similar to the radiotherapy machine **141** of **FIG. 1****.** The radiotherapy machine **241** may further include a display screen **226** positioned on or within the gantry **224.** The display screen **226** may be configured to display for view to a user (e.g., a technician, patient, and/or a medical practitioner) relevant information related to the radiotherapy procedure before, during, and/or after the radiotherapy procedure.

The radiotherapy machine **241** may also include an imager **270** and an emitter **260** for administering the radiotherapy to a patient. The radiotherapy system **240** may additionally include a couch **222,** upon which the patient may be placed during the administration of the radiotherapy. In some embodiments, the couch **222** may be translated laterally relative to the radiotherapy machine **241** (e.g., along the center axis **225**) and/or rotated axially relative to the center axis **225** of the radiotherapy machine **241.** Likewise, in some embodiments, the radiotherapy machine **241** may be rotated axially about the center axis **225,** and relative to the couch **222,** before, during, and/or after the administration of the radiotherapy treatment.

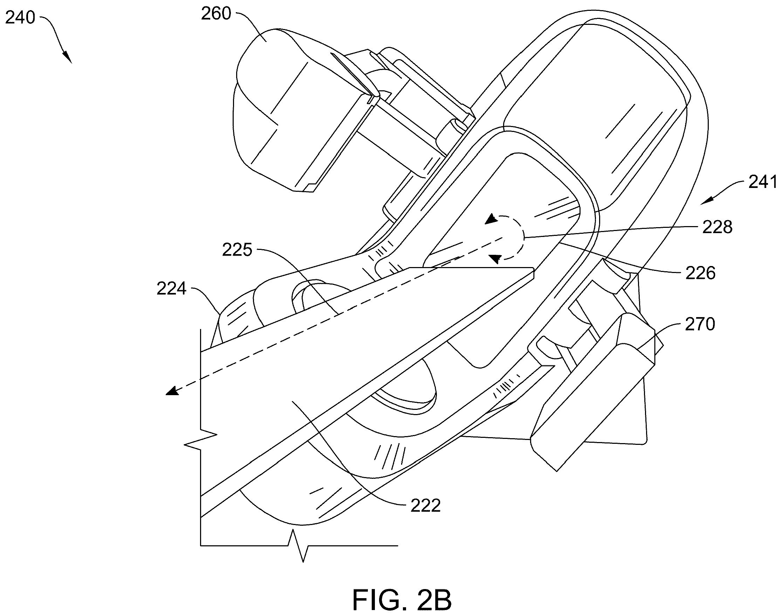

For example, as illustrated in **FIG. 2B****,** the radiotherapy machine **241** is shown in a rotated position about the center axis **225.** Relative to the default position of the radiotherapy machine **241** (as shown in **FIG. 2A**), the radiotherapy machine **241** in **FIG. 2B** is shown at a position that is rotated about 135.5° counter-clockwise. Because the display screen **226** is coupled to the gantry **224** of the radiotherapy machine **241,** the display screen **226** is also rotated from a default, upright position (as shown in **FIG. 2A**) at about 135.5° counterclockwise.

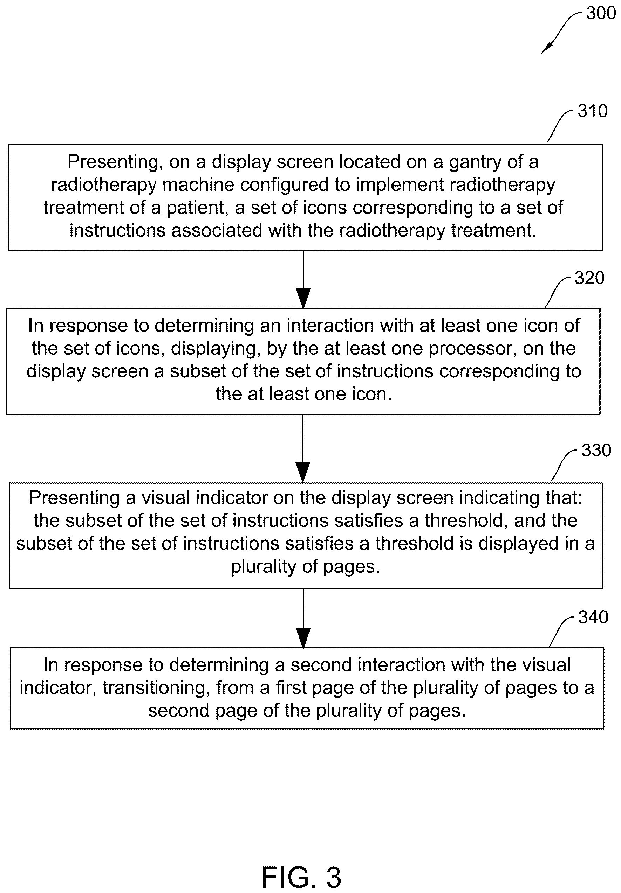

**FIG. 3** is a flowchart of an example method **300** for displaying one or more icons on a display screen coupled to a radiotherapy machine. The method **300** may be executed by one or more processors described herein, such as the analytics server **110,** the pendant **142,** the system administrator computer **150,** the radiotherapy system **140,** the radiotherapy machine **141,** etc. The one or more processors may execute instructions stored on a computer-readable, non-transitory medium, which may cause the one or more processors to execute steps of the method **300.** While the steps of the method **300** are shown in a specific order, it should be understood that the method **300** may comprise more, fewer, and/or different steps than those depicted in **FIG. 3****.** Likewise, the order of the steps of the method **300** is shown in **FIG. 3** for illustrative purposes. However, it is understood that the steps of method **300** may be performed in any number of configurations or orders without departing from the scope of the systems and methods described herein.

At step **310,** the one or more processors may present, on a display screen located on a gantry of a radiotherapy machine configured to implement radiotherapy treatment of a patient, a set of icons corresponding to a set of instructions associated with the radiotherapy treatment and/or setup.

In some embodiments, the one or more processors may present, on a display screen located on the gantry of a radiotherapy machine, a set of icons corresponding to a set of tasks needed to implement/facilitate a patient's radiotherapy treatment and/or setup. As discussed herein, e.g., **FIGS. 2A-B**, the display screen may be integrated into the gantry and may serve as an intuitive and interactive interface for operators, providing task-specific visual elements that guide the execution of various stages of the treatment and/or setup process. Each icon may represent a distinct instruction or task within the treatment workflow, such as patient alignment, equipment setup, dose calibration, or verification of treatment parameters.

In some embodiments, the one or more processors dynamically update the display to ensure the presented icons are relevant to the various stages of the radiotherapy treatment and/or setup. In some embodiments, the one or more processors may generate a set of tasks for configuring a radiotherapy treatment and/or setup for a patient, with each task associated with a specific stage of the treatment and/or treatment setup. This structured and workflow-oriented approach ensures that the radiotherapy treatment and/or setup process is methodical and tailored to the patient's specific needs. To initiate the process, the processors may retrieve a radiotherapy file (RT file) associated with the patient, which contains critical treatment data. The RT file serves as a comprehensive repository of information necessary for setting up, planning, executing, and/or monitoring the radiotherapy treatment. It may include patient identifiers such as name and demographic details, imaging data (e.g., CT scans, MRIs, x-rays), and detailed treatment plans, including beam trajectories, dose levels, arc information, and radiotherapy types. Additionally or alternatively, it specifies target and non-target organ data to ensure precise radiation delivery while minimizing exposure to healthy tissues.

The RT file may also contain patient-specific attributes like physical characteristics (e.g., height, weight, BMI), medical history, and session-specific details such as prior couch positioning adjustments. It may include required accessory details, like immobilization devices or bolus materials, enabling consistency across treatment sessions. Leveraging this file, the processors dynamically generate workflow tasks (for example, treatment and/or treatment setup workflow tasks), populate the GUI with task-relevant values, and provide real-time updates as tasks are completed. These tasks correspond to various stages of the radiotherapy treatment and/or setup workflow, such as patient alignment, machine calibration, accessory verification, dose administration, and post-treatment analysis. For instance, during the alignment stage, tasks may guide the operator in adjusting the couch and gantry to achieve proper alignment relative to the machine's isocenter. As tasks are completed, the processors or servers update the icons displayed on the GUI to reflect task status, ensuring seamless progression through the workflow. For example, processors may detect a completed task, such as couch movement, and update the corresponding icon, or operators may input task completion using a pendant, prompting the processors to adjust the GUI accordingly.

In some embodiments, the icons may be designed with visual attributes, such as color, shape, or animations, that reflect the status of each instruction, providing operators with immediate feedback on task completion or required actions. This dynamic and task-specific presentation enhances usability, reduces cognitive load, and ensures a streamlined and error-resistant workflow for delivering precise and effective radiotherapy treatment and/or radiotherapy setup.

After the tasks to be performed by the operator have been identified, the one or more processors may display the set of icons. In some embodiments, the set of icons may be radially arranged. That is, the icons may be radially arranged around a center point on the display screen, allowing for uniform distribution and simultaneous visibility, regardless of the screen's orientation or the gantry's position. This radial configuration may enhance accessibility and usability, enabling operators to efficiently locate and interact with icons without diverting attention from the patient or machine. In some embodiments, the icons can also be grouped based on predefined attributes, such as workflow stage or type of information, with customization options available to adapt groupings to the preferences of different operators or clinics.

Furthermore, the icons are dynamically interactive, responding to changes in the treatment environment and user inputs. For instance, once the patient alignment is verified, the corresponding icon may change color or display a checkmark to indicate completion, while the next icon highlights instructions for dose calibration. These dynamic and customizable features provide operators with an intuitive and efficient interface that reduces cognitive load, improves workflow precision, and enhances the overall radiotherapy treatment process.

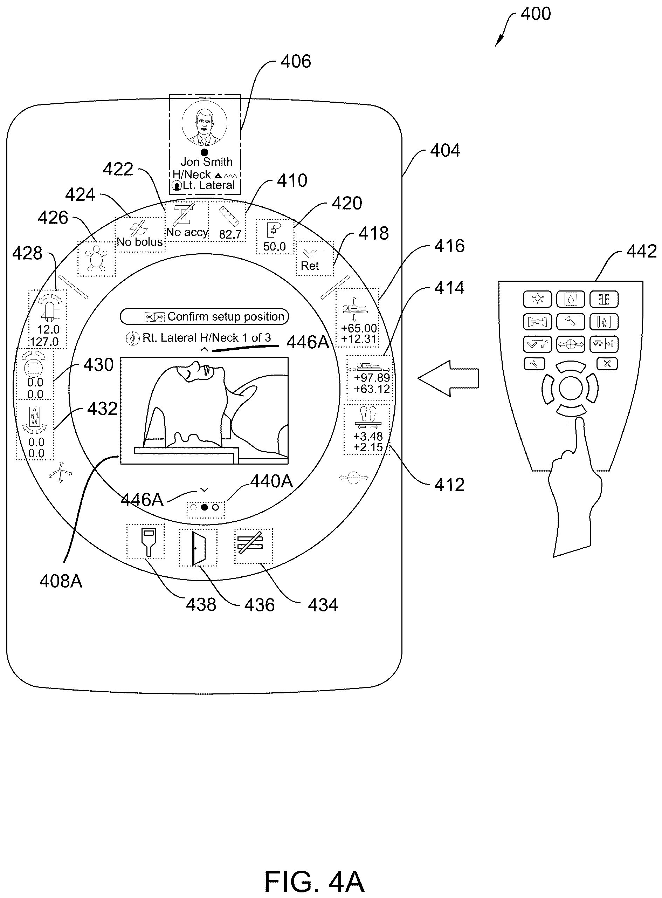

Referring now to **FIG. 4A****,** a display of the icons on a display screen of a radiotherapy machine is presented, in accordance with one embodiment. The set of icons, in this embodiment **400,** are presented on a display screen **404** (similar to the display screen **226**) located on a gantry of a radiotherapy machine. As depicted, the icons **406-432** are displayed radially on the display screen **404,** though in other embodiments, the arrangement may be different. The display screen may also include a center portion **408A-C** that can display additional data needed or otherwise associated with different icons.

As depicted, the set of displayed icons includes an icon **406** that may include the patient's personally identifiable information, such as the patient's image, name, diagnoses, and other information that can identify the patient.

Different groupings of icons (or sometimes individual icons) may be arranged in accordance with a predefined order that may or may not correspond to the workflow of the patient's radiotherapy treatment. For instance, in one embodiment, different tasks to be performed may be associated with a defined order. As a result, their corresponding icons may be arranged in that order.

In other embodiments, such as the depicted embodiment, the icons may be arranged in accordance with a grouping of the tasks. For instance, icons may be arranged based on whether they are associated with a patient setup category, radiotherapy accessory category, radiotherapy machine attribute category, rotational parameter category, interlocks, or a system status category. As depicted, the set of icons may be divided into three groups where the groups indicate a cluster of tasks to be performed. The cluster of tasks may have a common attribute and may be clustered based on the common attribute. For instance, one grouping or cluster of icons may indicate adjustments to be made to the patient (e.g., patient alignment) and another grouping of the icons may be directed to machine adjustment.

A first grouping of the icons may include patient alignment indicators (e.g., icons **412, 414,** and **416**). The icon **412** may indicate whether the patient needs to be laterally moved (e.g., moved to the left or right). Additionally, the set of icons may include an icon **414** that indicates whether the patient should be moved on the couch (e.g., toward the radiotherapy machine or away from the machine). Additionally, the set of icons may include an icon **416** that indicates whether the patient on the couch should be moved vertically (e.g., whether the couch should be moved up or down).

A second grouping of icons may include machine/treatment accessories and may include icons **410, 418, 420, 422,** and **424.** The icon **410** may correspond to a distance indicator measuring/indicating the distance between the radiation source and the patient's skin (source-to-skin distance, SSD). Additionally, the set of icons may include an icon **418** that indicates whether an imager of the machine is extended. Additionally, the set of icons may include an icon **420** that indicates the status of the imagers of the machine (e.g., how far the imagers are from the machine). Additionally, the set of icons may include an icon **422** that indicates whether an accessory (and if so, which accessory) is needed to treat the patient. Additionally, the set of icons may include an icon **424** that indicates whether a bolus is needed to treat the patient. Additionally, the set of icons may include an icon **426** that indicates the patient orientation (e.g., whether the patient should be on their back with their head toward the machine or otherwise). Additionally, the set of icons may include an icon **428** that indicates whether any adjustment to the gantry is needed. The icon **428** may include arrows indicating which direction the gantry should be turned or otherwise moved towards. In some embodiments, the icon **428** may also include an angle value indicating a desired position of the gantry.

Another grouping of the icons may include machine attribute icons that correspond to tasks related to adjusting the radiotherapy machine. This group may include icons **428, 430,** and **432.** The icon **430** may indicate whether any adjustments are needed to be made to the collimator. In some embodiments, the icon **430** may include a map of the collimator opening. Additionally, the set of icons may include an icon **432** indicating a couch rotation angle (yaw).

In some embodiments, system administrators or operators may have the capability to adjust any attribute of the icons displayed on the display screen, including visual attributes such as color, font style, size, line thickness, shading, and the like. This flexibility allows the icons to be modified to align with institutional branding, operator preferences, or accessibility requirements. For instance, line thickness or font size can be increased to enhance visibility for users with visual impairments. These adjustments ensure that the interface remains both functional and user-friendly, tailored to meet the specific needs of the clinical setting.

The position of the icons on the display screen can also be adjusted by system administrators or operators to optimize usability and align with operator workflows. Icons can be repositioned within the radial arrangement or moved to alternative layouts, ensuring that the most frequently accessed icons are conveniently located. This customization enables the interface to adapt to specific clinic requirements or individual user preferences, improving efficiency during radiotherapy procedures. For instance, a clinic focusing on particular types of treatments may prioritize the placement of certain icons for quick access, minimizing navigation time and enhancing operational precision.

In some embodiments, a system administrator or an operator can be authorized to add new ones to better reflect the workflow or accommodate updates to the radiotherapy process. This capability ensures that the interface remains relevant and streamlined, reducing visual clutter and focusing only on the tasks and information pertinent to the specific clinical scenario. For example, an icon can be added to the set of icons that indicates any combination of one or more of: whether a door of the treatment room is open, whether a predicted collision is going to occur, whether the pendant is properly connected to the radiotherapy machine, and the like.

The set of icons may also include an icon **438** indicating whether a pendant is properly connected to the radiotherapy machine (e.g., whether the pendant is not properly placed in its docking position at the end of the couch). In some embodiments, the treatment may be blocked if the pendant is not docked. The set of icons may also include the icon **436** indicating whether the treatment room door has been properly closed and/or icon **434** indicating whether the machine parameters are or are not in the planned (e.g., required) position for treatment.

The icons displayed may be interactive, such that the operator can move along the icons and select (e.g., click, press, touch, or otherwise interact with different icons), e.g., using a pendant of the radiotherapy machine, such as the pendant **442.** For instance, the operator may use one or more buttons on the pendant **442** to navigate through the displayed icons and use another button to make a selection.

The depiction of the pendant **442** is not limiting to the embodiments discussed herein. Specifically, the pendant **442** is not the only way the operator can interact with the icons displayed on the display screen. In other embodiments, the operator can use various alternative input elements to navigate and interact with the system. For example, a touchscreen interface integrated into the display screen (or other places within the radiotherapy machine) may allow direct selection of icons with a simple tap or swipe gesture. Additionally, a controller can be used to move a cursor or highlight different icons for selection. Some embodiments may provide a foot pedal interface hands-free control, enabling the operator to cycle through icons or confirm selections while keeping their hands free for patient positioning. In some implementations, a gesture recognition system may detect hand movements to trigger specific actions, enhancing accessibility. Furthermore, a voice command system may be employed, allowing the operator to verbally navigate the interface and select icons without any physical interaction.

Referring back to **FIG. 3****,** at step **320,** the one or more processors may, in response to determining an interaction with at least one icon of the set of icons, display on the display screen a subset of the set of instructions corresponding to the at least one icon.

The one or more processors are configured to dynamically respond to interactions with icons displayed on the display screen. As discussed herein, the operator can navigate through different icons displayed, using a pendant or any other input element (e.g., mouse, keyboard, touch screen, and the like). Using the input element, the operator may click (or otherwise interact with) an icon. Upon detecting an interaction with at least one icon from the set of icons, the one or more processors may display, on the same screen, at least a subset of instructions corresponding to the selected icon. This functionality ensures that operators are provided with detailed, context-specific guidance for completing the task or action represented by the icon, enhancing precision and usability during the radiotherapy treatment workflow.

The instructions may provide the information that is needed to accomplish the tasks associated with the selected icon. By presenting only the instructions relevant to the selected task, the system minimizes visual clutter and provides the operator with focused, actionable information, ensuring efficient task execution and seamless progression through the radiotherapy and/or radiotherapy setup workflow. This dynamic interaction capability further enhances the adaptability and precision of the system in delivering personalized and accurate radiotherapy treatments and/or radiotherapy treatment setup.

Moreover, the operator can simultaneously view the status of other tasks and icons. For instance, when an operator clicks on an alignment icon, the processors can display the information needed regarding how to align the patient. Advantageously, the operator can view, at the same time, whether any bolus or accessories are needed. In contrast to conventional methods where the information was provided to the operators in a segmented manner (and the operator would need to stop the alignment process to gather the needed accessory), the display methods discussed herein allow the operator to ensure that the bolus and accessories (if needed) are present before starting the alignment process. Accordingly, the operator is no longer required to stop the alignment process, retrieve the needed bolus/accessory, and restart the process.

Referring back to **FIG. 4A****,** as icon(s) are selected (e.g., using the pendant 442), the center portion **408A** of the display screen may be dynamically populated with detailed information and/or actionable instructions necessary to accomplish the tasks associated with the selected icon(s). Though the depicted embodiment illustrates the information displayed in a center portion, in some embodiments, the information may be displayed in another portion of the display screen. The location, size, font, and other visual attributes of the information displayed may be customizable by the operator or a system administrator. This dynamic content displayed may provide the operator with relevant and context-specific data without overwhelming the interface with unnecessary information. Moreover, the operator is not required to navigate to (or otherwise be directed to) another page.

For example, as depicted, the operator has selected a patient alignment/positioning icon. As a result, the center portion **408A** may be dynamically populated with any information (e.g., video, animation, text, and the like) needed to illustrate how the patient should be aligned for the patient's treatment. In the depicted embodiment, an image of a patient may be displayed in the center portion **408A** that illustrates how the patient should be adjusted. The center portion **408A** may also include text indicating that the patient's head/neck should be adjusted and may also include appropriate alignment angles as well.

Referring back to **FIG. 3****,** at step **330,** the one or more processors may present a visual indicator on the display screen indicating that the subset of the set of instructions satisfies a threshold, and the subset of the set of instructions is displayed in a plurality of pages.

The visual indicator may be designed to inform the operator that a subset of the set of instructions satisfies a predefined threshold. As used herein, the threshold may correspond to any attribute that is defined by a system administrator or an operator. For instance, the threshold may correspond to a number of instructions for the selected icon. In another example, the threshold may indicate a predefined progression of instructions that are designed to be separately and/or consecutively displayed. For example, when the subset of instructions exceeds the display capacity of a single page, the one or more processors organize and display the instructions across a plurality of pages, ensuring that all relevant information is accessible without overwhelming the interface or compromising usability.

In some embodiments where the subset of instructions contains numerous detailed steps, such as alignment adjustments, gantry configuration, or dose calibration, the one or more processors may distribute these steps across multiple pages. Moreover, the one or more processors may display a visual indicator informing the operator that the instructions are displayed on multiple pages. Non-limiting examples of a visual indicator may include visual navigation cues, such as pagination icons, scrolling indicators, toggle icons, or a progress bar, and the like. The visual indicator may allow the operator to seamlessly navigate between pages while ensuring clarity and task focus. For instance, the indicator may convey the number of pages and the position of the current page (among the set of pages) being displayed. This functionality ensures that the interface remains efficient and user-friendly, even when managing complex or large sets of instructions, ultimately improving workflow efficiency and treatment accuracy.

Referring back to **FIG. 4A****,** the one or more processors may determine that the selected icon includes more information than can be displayed on one page. As a result, the one or more processors may display the visual indicator **446A** indicating that the information displayed in the center portion **408A** includes multiple pages. The visual indicator **446A** indicates that the information associated with the selected icon is displayed in multiple pages.

Referring back to **FIG. 3****,** at step **340,** the one or more processors may, in response to determining a second interaction with the visual indicator, transition from a first page of the plurality of pages to a second page of the plurality of pages. The one or more processors may enable navigation between pages of a multi-page interface on the display screen. In response to determining a second interaction with the visual indicator, the one or more processors may transition from a first page of a plurality of pages to a second page. For example, during the configuration of a radiotherapy treatment, the first page may display instructions related to patient alignment, such as numerical adjustments and visual alignment guides. If the operator interacts with the visual indicator, such as clicking a pagination button or swiping a touch-sensitive area, the one or more processors may transition to a second page that contains additional instructions or information.

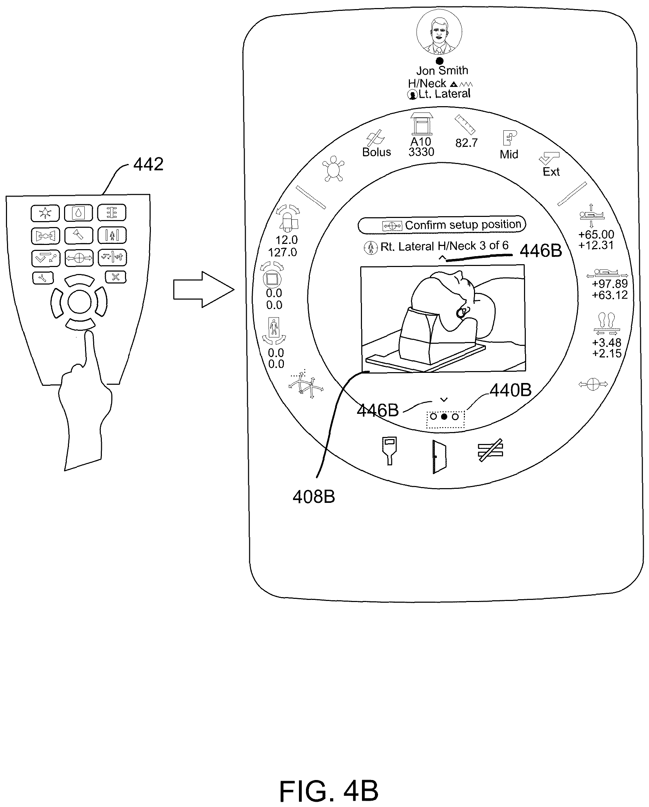

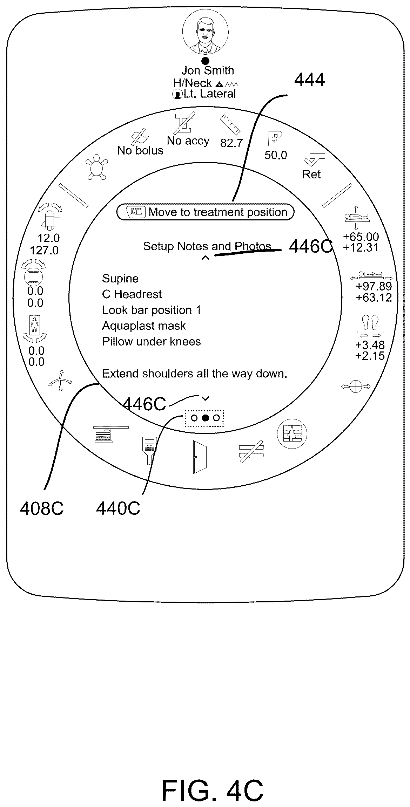

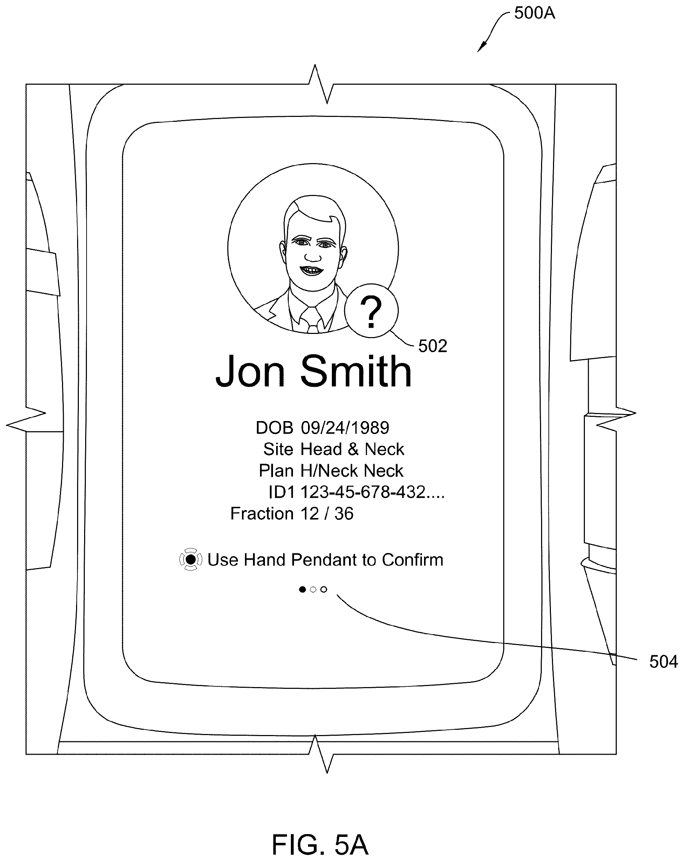

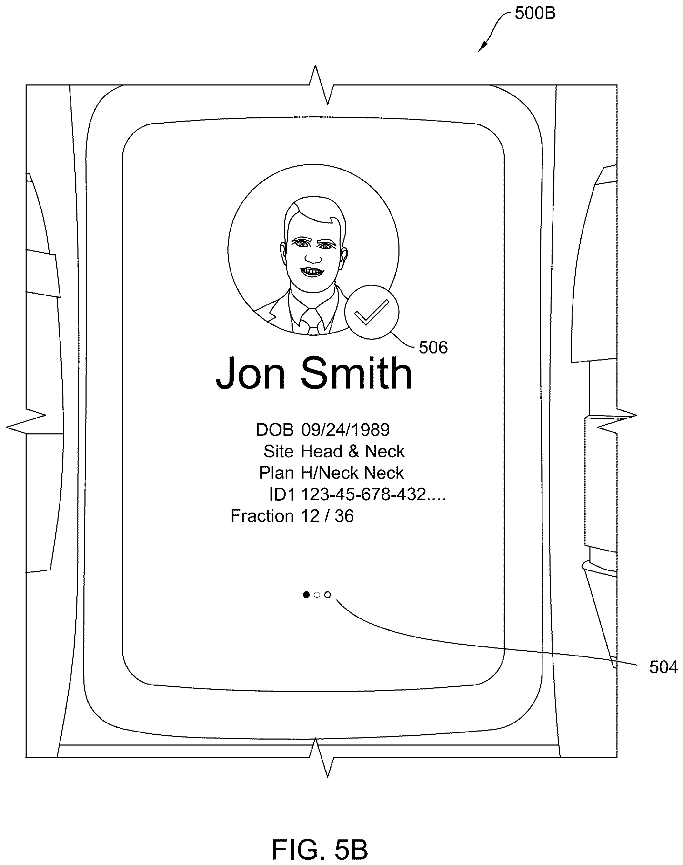

Referring back to **FIG. 4A****,** when the one or more processors determine that the operator has interacted with the visual indicator **446A,** the one or more processors may transition the center portion **408A** to a subsequent page (within the series of pages that includes the instructions). Accordingly, the one or more processors may dynamically update the center portion **408A** to center portion **408B,** as depicted in **FIG. 4B****.** The center portion **408B** may represent a "next" or "subsequent" page that includes instructions that are subsequent to the instructions displayed in the center portion **408A.** The operator may use the visual indicator **446B** to also navigate to a third page, as depicted in **FIG. 4C** or go back to the first page (**FIG. 4A**). In **FIG. 4C****,** the visual indicator is updated to indicate that the operator is viewing the third page. Moreover, the center portion **408C** is also updated to display the subsequent instructions/information. The center portion **408C** may also include the interactive element **444** allowing the operator to navigate to another icon/stage/task. Moreover, the operator can use the visual indicators **446C** to navigate back to the second page depicted in **FIG. 4B****.** At any time, the operator can use the visual indicators 440A-C to move back and forth to the pages depicted in **FIGS. 5A-B**.

Though the embodiment depicted in **FIGS. 4A-C** depict a center portion **408A-C** that transitions to different pages, the methods and systems discussed herein are not limited to a specific part or portion of the screen transitioning. The content (e.g., instructions associated with different icons) can be displayed anywhere on the display screen. For instance, the content can be displayed on the top center (or right or left) of the display screen, allowing the operator to view the guidance prominently while maintaining visibility of other icons on the lower sections. Alternatively, the content can be displayed on the bottom portion or either side of the display screen. In some embodiments, the content may be displayed in the entire display screen and the entire display screen may transition to different pages to display the relevant content, temporarily hiding other elements to focus the operator's attention on the specific task at hand.

In some embodiments, the different pages included within the group of pages may be predefined by a system administrator. For instance, a system administrator may define a number of pages and a corresponding subset of the instruction and/or radiotherapy information to be included within each page along with an order of the pages. In a non-limiting example, when an icon is interacted with by the operator, the one or more processors may retrieve the defined number and a sequential order (sequence) of pages along with the information to be displayed on each page. Then the one or more processors display the pages sequentially in accordance with the defined order.

In some embodiments, a patient identification GUI may be presented before other GUIs or before any other icons are displayed. For instance, and referring to **FIG. 5A****,** a page **500A** is presented to facilitate patient identification as part of the radiotherapy workflow discussed herein.

As depicted, the page **500A** prominently displays patient-specific information to ensure accurate treatment administration. On the page **500A,** the one or more processors may display a visual representation of the patient, such as a photograph, along with their name (e.g., "Jon Smith"). The page **500A** may also include additional patient details, such as the patient's date of birth (DOB), treatment site (e.g., "Head & Neck"), treatment plan (e.g., "H/Neck Neck"), a unique patient identifier (e.g., "ID1 123-45-678-432..."), and the current fraction of treatment being administered relative to the total prescribed sessions (e.g., "Fraction 12/36").

This structured presentation of information ensures that operators can quickly verify patient identity and treatment details, reducing the risk of misidentification or treatment errors. In some embodiments, the page **500A** may include navigation indicators, such as dots **504** at the bottom of the screen, to signify that multiple pages of patient information can be accessed by swiping or selecting additional options.

In some embodiments, the dots **504** may signify the presence of multiple pages, each tailored to different aspects of the radiotherapy workflow. Specifically, by navigating different pages (via interacting with the dots **504**), the operator may access different types of pages where each page is directed toward a different aspect of the radiotherapy and/or radiotherapy setup workflow. For example, the "patient pages" may display patient-specific information, such as name, date of birth, treatment site, and session details, ensuring clear and accurate identification during the setup process. A non-limiting example of a patient page is the page **500.** In contrast, other pages within the same user interface may adopt a different layout and content, focusing on machine parameters, therapist workflows, or treatment configurations. Non-limiting examples of other pages may include the pages depicted in **FIGS. 4A-C** where these pages are dedicated to displaying treatment-related information (e.g., how to set up the patient and/or the machine by adjusting the couch/gantry).

This toggle approach between different pages allows for the concept of distinct screen archetypes within a unified user interface, enabling seamless transitions between different pages where different categories of radiotherapy data is displayed. As used herein, an archetype may refer to a category of radiotherapy data that is displayed. For instance, one archetype or category of data may refer to pages where patient-oriented data is displayed. Another non-limiting example of an archetype of category of radiotherapy data may include pages where machine or therapist-oriented data/views are displayed. The systems and methods discussed herein allow for the user to switch back and forth and access different categories of data.

By navigating between pages, the interface can dynamically adapt its purpose and character to suit the current stage of the workflow, such as patient identification, alignment, machine parameter adjustments, and the like. In a non-limiting example, the operator may navigate back and forth between these different page types using the depicted dots **504.** For instance, after confirming the patient identity, the operator may use the pendant to interact with the depicted dots **504** and navigate to the user interface depicted in **FIG. 4A** (e.g., embodiment **400**), where the operator can start preparing the patient and the radiotherapy machine for treatment. At any time, the operator can then navigate back to the page **500** (patient page) if needed. The operator can then interact with the dots **446A-C** in order to navigate back to the pages **500A-B.** In this way, the dots displayed at the bottom of the page can be used to navigate through different types of user interfaces (e.g., from patient-based UIs to machine-related UIs).

The dots **504** provide an intuitive navigation mechanism, allowing operators to switch between these archetypes as needed, ensuring that the user interface remains flexible, purpose-driven, and optimized for the specific requirements of each workflow step. This adaptability enhances operational efficiency, minimizes cognitive load, and supports a streamlined radiotherapy process.

The page **500A** allows the operator to confirm the identity of the patient using various input elements discussed herein, such as by interacting with a button on the pendant. After the patient identity is confirmed, the one or more processors may display an indicator on the page **500A** using one or more methods discussed herein. In one embodiment, the indicator may be a color-coded check mark that can appear on the page **500A** in an animation style. For instance, the page **500A** includes an indicator **502,** a question mark usually in yellow to indicate that the patient identity is not confirmed. When the patient identity is confirmed, the one or more processors may transition to a page **500B.** The page **500B** includes an indicator **506** which displays a check mark, usually in green, to indicate that the patient identity has been confirmed. The transition between the indicators **502** and **506** may be visually highlighted, such as using an animation style or a pulsating visual indicator, such that the change from the unconfirmed patient identity (indicator **502**) to a confirmed patient identity (indicator **506**) is easily identified by the operator.

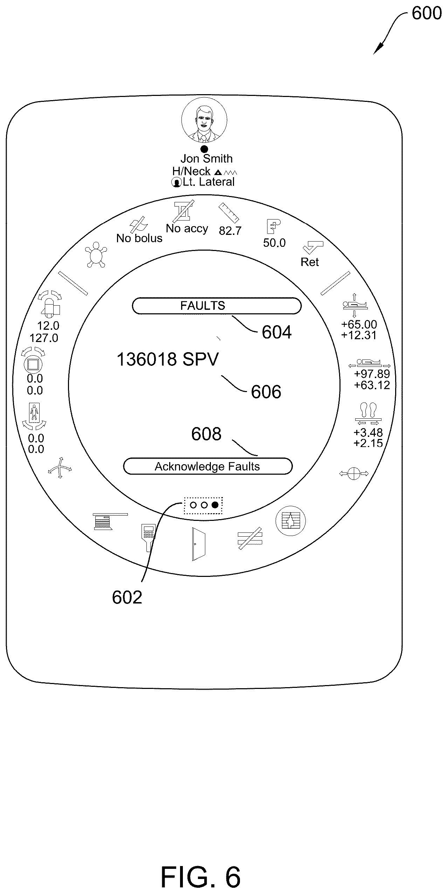

In some embodiments, the operator can use the visual indicators discussed herein (dots **504, 602,** and/or the visual indicators **440A-C**) to navigate to a dedicated page. For instance, the operator can predefine a particular page (e.g., patient alignment or machine alignment) and automatically navigate to that defined page (as opposed to navigating to the page **400** depicted in **FIG. 4A** and then clicking on the pertinent icon). In some embodiments, a dedicated page may display the faults or warnings. For instance, using the visual indicators discussed herein, the operator may navigate to the page **600** (**FIG. 6**) that indicates warnings and "faults." As used herein, the one or more processors may periodically analyze various patient and machine parameters and determine if any of the instructions are not followed (e.g., a machine setting does not match with the machine setting that is needed before the patient's treatment can start). As a result, the one or more processors discussed herein can display the page **600** that indicates which faults (**604**) or errors need to be rectified. As depicted, the page **600** may indicate the faults/errors (e.g., faults **606** that include a code associated with the fault) and include an input element **608** that requires the operator to acknowledge those faults.

In some embodiments, the page **600** may not be a separate or dedicated page to display faults or errors. Accordingly, the page **600** may not be reached using the navigational paradigm discussed herein (e.g., using the visual indicators **440A-C** and/or dots **504**). Instead, the content of the page **600** (e.g., faults and other important messages regarding errors) may be displayed as an overlay displayed in conjunction with (over) the currently selected page, similar to a popup message. This approach ensures that critical alerts interrupt the operator's workflow only when necessary and demand immediate attention without requiring the operator to navigate away from their current interface. Such messages may be triggered either by user actions, such as resetting the patient position, or by the system itself, such as when a fault condition is detected. By presenting these messages as overlays, the system maintains workflow efficiency while ensuring that urgent issues are promptly addressed without unnecessary navigation disruptions.

### Example clauses

Further aspects of these teachings are provided by the subject matter of the following clauses.

Clause 1. A system comprising a computer-readable medium having non-transitory instructions, that when executed, cause at least one processor to: present, on a display screen located on a gantry of a radiotherapy machine configured to implement radiotherapy treatment of a patient, a set of icons corresponding to a set of instructions associated with the radiotherapy treatment; in response to determining an interaction with at least one icon of the set of icons, display on the display screen a subset of the set of instructions corresponding to the at least one icon; present a visual indicator on the display screen indicating that the subset of the set of instructions satisfies a threshold and is displayed in a plurality of pages; and in response to determining a second interaction with the visual indicator, transition from a first page of the plurality of pages to a second page of the plurality of pages while displaying the set of icons on both the first page and the second page.

Clause 2. The system of clauses 1, wherein the threshold corresponds to a number of instructions of the subset of the set of instructions.

Clause 3. The system of clauses 1-2, wherein the threshold corresponds to a sequential order of the subset of the set of instructions.

Clause 4. The system of clauses 1-3, wherein the visual indicator conveys a number of pages of the plurality of pages.

Clause 5. The system of clauses 1-4, wherein the second interaction indicates a particular page of the plurality of pages being displayed.

Clause 6. The system of clauses 1-5, wherein the first page displays a different category of radiotherapy data than the second page.

Clause 7. A system comprising: a radiotherapy machine having a display screen; and at least one processor in communication with the display screen, the at least one processor configured to: present, on the display screen located on a gantry of the radiotherapy machine configured to implement radiotherapy treatment of a patient, a set of icons corresponding to a set of instructions associated with the radiotherapy treatment; in response to determining an interaction with at least one icon of the set of icons, display on the display screen a subset of the set of instructions corresponding to the at least one icon; present a visual indicator on the display screen indicating that the subset of the set of instructions satisfies a threshold and is displayed in a plurality of pages; and in response to determining a second interaction with the visual indicator, transition from a first page of the plurality of pages to a second page of the plurality of pages while displaying the set of icons on both the first page and the second page.

Clause 8. The system of clause 7, wherein the threshold corresponds to a number of instructions of the subset of the set of instructions.

Clause 9. The system of clauses 7-8, wherein the threshold corresponds to a sequential order of the subset of the set of instructions.

Clause 10. The system of clauses 7-9, wherein the visual indicator conveys a number of pages of the plurality of pages.

Clause 11. The system of clauses 7-10, wherein the second interaction indicates a particular page of the plurality of pages being displayed.

Clause 12. The system of clauses 7-11, wherein the processor is further configured to revise a visual attribute of the at least one icon.

Clause 13. The system of clauses 7-12, wherein the first page displays a different category of radiotherapy data than the second page.

Clause 14. A method comprising: presenting, by at least one processor on a display screen located on a gantry of a radiotherapy machine configured to implement radiotherapy treatment of a patient, a set of icons corresponding to a set of instructions associated with the radiotherapy treatment; in response to determining an interaction with at least one icon of the set of icons, displaying, by the at least one processor, on the display screen a subset of the set of instructions corresponding to the at least one icon; presenting, by the at least one processor, a visual indicator on the display screen indicating that the subset of the set of instructions satisfies a threshold and is displayed in a plurality of pages; and in response to determining a second interaction with the visual indicator, transitioning, by the at least one processor, from a first page of the plurality of pages to a second page of the plurality of pages while displaying the set of icons on both the first page and the second page.

Clause 15. The method of clause 14, wherein the threshold corresponds to a number of instructions of the subset of the set of instructions.

Clause 16. The method of clauses 14-15, wherein the threshold corresponds to a sequential order of the subset of the set of instructions.

Clause 17. The method of clauses 14-16, wherein the visual indicator conveys a number of pages of the plurality of pages.

Clause 18. The method of clauses 14-17, wherein the second interaction indicates a particular page of the plurality of pages being displayed.

Clause 19. The method of clauses 14-18, further comprising: revising, by the at least one processor, a visual attribute of the at least one icon.

Clause 20. The method of clauses 14-19, wherein the first page displays a different category of radiotherapy data than the second page.

Clause 21: A computer program comprising instructions which, when the program is executed by a computer, cause the computer to carry out the method of any of clauses 14-20.

Clause 22: A computer-readable medium comprising a set of instructions, that when executed by one or more processors, cause the one or more processors to carry out the method of any of clauses 14-20.

The presentation of information on the display screen, the user interaction with the display screen and/or the GUI, may have the technical effect of assisting the user in performing a radiotherapy setup workflow by means of a continued and/or guided process of human-machine interaction. The systems discussed herein may be configured to assist the user in performing a radiotherapy workflow and/or a radiotherapy setup workflow, by means of a continued and/or guided process of human-machine interaction.

In this disclosure, the term "radiotherapy treatment" may refer to radiotherapy treatment and/or radiotherapy treatment setup. The method steps may refer to treatment setup, while the systems may be configured to perform treatment and/or treatment setup.

The foregoing method descriptions and the process flow diagrams are provided merely as illustrative examples and are not intended to require or imply that the steps of the various embodiments must be performed in the order presented. The steps in the foregoing embodiments may be performed in any order. Words such as "then," "next," etc. are not intended to limit the order of the steps; these words are simply used to guide the reader through the description of the methods. Although process flow diagrams may describe the operations as a sequential process, many of the operations can be performed in parallel or concurrently. In addition, the order of the operations may be re-arranged. A process may correspond to a method, a function, a procedure, a subroutine, a subprogram, and the like. When a process corresponds to a function, the process termination may correspond to a return of the function to a calling function or a main function.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the present invention.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

The actual software code or specialized control hardware used to implement these systems and methods is not limiting of the invention. Thus, the operation and behavior of the systems and methods were described without reference to the specific software code being understood that software and control hardware can be designed to implement the systems and methods based on the description herein.

When implemented in software, the functions may be stored therein as one or more instructions or code on a computer-readable, non-transitory medium or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the present invention. Various modifications to these embodiments will be readily apparent to those skilled in the art, and the generic principles defined herein may be applied to other embodiments without departing from the scope of the invention. Thus, the present invention is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope being indicated by the following claims.

## Claims

1. A computer-readable medium having non-transitory instructions, that when executed, cause a processor to:
present, on a display screen coupled to a radiotherapy machine configured to implement radiotherapy treatment of a patient, a set of icons corresponding to a set of instructions associated with the radiotherapy treatment;
in response to determining an interaction with at least one icon of the set of icons, display on the display screen a subset of the set of instructions corresponding to the at least one icon;
present a visual indicator on the display screen indicating that the subset of the set of instructions satisfies a threshold and is displayed in a plurality of pages; and
in response to determining a second interaction with the visual indicator, transition from a first page of the plurality of pages to a second page of the plurality of pages while displaying the set of icons on both the first page and the second page.

2. The computer-readable medium of claim 1, wherein the threshold corresponds to a number of instructions of the subset of the set of instructions.

3. The computer-readable medium of claim 1 or claim 2, wherein the threshold corresponds to a sequential order of the subset of the set of instructions.

4. The computer-readable medium of any preceding claim, wherein the visual indicator conveys a number of pages of the plurality of pages.

5. The computer-readable medium of any preceding claim, wherein the second interaction indicates a particular page of the plurality of pages being displayed;
and/or:
wherein the first page displays a different category of radiotherapy data than the second page.

6. A system comprising:
a radiotherapy machine having a display screen; and
at least one processor in communication with the display screen, the at least one processor configured to:
present, on the display screen coupled to the radiotherapy machine configured to implement radiotherapy treatment of a patient, a set of icons corresponding to a set of instructions associated with the radiotherapy treatment;
in response to determining an interaction with at least one icon of the set of icons, display on the display screen a subset of the set of instructions corresponding to the at least one icon;
present a visual indicator on the display screen indicating that the subset of the set of instructions satisfies a threshold and is displayed in a plurality of pages; and
in response to determining a second interaction with the visual indicator, transition from a first page of the plurality of pages to a second page of the plurality of pages while displaying the set of icons on both the first page and the second page.

7. The system of claim 6, wherein the threshold corresponds to a number of instructions of the subset of the set of instructions.

8. The system of claim 6 or claim 7, wherein the threshold corresponds to a sequential order of the subset of the set of instructions.

9. The system of any of claim 6 to claim 8, wherein the visual indicator conveys a number of pages of the plurality of pages;
and/or:
wherein the second interaction indicates a particular page of the plurality of pages being displayed.

10. The system of any of claim 6 to claim 9, wherein the processor is further configured to revise a visual attribute of the at least one icon;
and/or:
wherein the first page displays a different category of radiotherapy data than the second page.

11. A method comprising:
presenting, by at least one processor on a display screen coupled to a radiotherapy machine configured to implement radiotherapy treatment of a patient, a set of icons corresponding to a set of instructions associated with the radiotherapy treatment;
in response to determining an interaction with at least one icon of the set of icons, displaying, by the at least one processor, on the display screen a subset of the set of instructions corresponding to the at least one icon;
presenting, by the at least one processor, a visual indicator on the display screen indicating that the subset of the set of instructions satisfies a threshold and is displayed in a plurality of pages; and
in response to determining a second interaction with the visual indicator, transitioning, by the at least one processor, from a first page of the plurality of pages to a second page of the plurality of pages while displaying the set of icons on both the first page and the second page.

12. The method of claim 11, wherein the threshold corresponds to a number of instructions of the subset of the set of instructions.

13. The method of claim 11 or claim 12, wherein the threshold corresponds to a sequential order of the subset of the set of instructions.

14. The method of any of claim 11 to claim 13, wherein the visual indicator conveys a number of pages of the plurality of pages;
and/or:
wherein the second interaction indicates a particular page of the plurality of pages being displayed.

15. The method of any of claim 11 to claim 14, further comprising: revising, by the at least one processor, a visual attribute of the at least one icon;
and/or:
wherein the first page displays a different category of radiotherapy data than the second page.
